# EUROPEAN PATENT SPECIFICATION

(11) **EP 3 697 467 B1**
(45) Date of publication and mention of the grant of the patent: **25.11.2026**
(21) Application number: 18796332.7
(22) Date of filing: 15.10.2018
(51) Int. Cl.: A61M 5/00, A61M 5/142

(54) **STERILIZATION ARRANGEMENT FOR DRUG DELIVERY DEVICE**
STERILISATIONSANORDUNG FÜR MEDIKAMENTENABGABEVORRICHTUNG
ARRANGEMENT DE STÉRILISATION POUR DISPOSITIF DE LIVRAISON DE MEDICAMENT

(30) Priority: 16.10.2017 US 201762572715 P
(43) Date of publication of application: 26.08.2020
(73) Proprietor: Becton, Dickinson and Company, Franklin Lakes, NJ 07417 (US)
(72) Inventor: FALKOVICH, Margarita, Paramus New Jersey 07652 (US)
(74) Representative: dompatent
(86) International application number: PCT/US2018/055865
(87) International publication number: WO 2019/079174

(56) References cited:
- WO-A1-2015/191478
- WO-A1-2017/142808
- WO-A2-2010/133698
- WO-A2-2015/081337
- CN-A- 105 999 482
- US-A1- 2005 047 975
- US-A1- 2013 317 422
- US-A1- 2016 367 767

## Description

### CROSS-REFERENCE TO RELATED APPLICATION

The present application claims priority to United States Provisional Application Serial No. 62/572,715, filed October 16, 2017, entitled "Sterilization Arrangement for Drug Delivery Device".

### BACKGROUND OF THE INVENTION

### Field of the Invention

The present disclosure relates generally to a drug delivery device and, in particular, to a sterilization arrangement for a drug delivery device.

### Description of Related Art

Various types of automatic injection or drug delivery devices have been developed to allow drug solutions and other liquid therapeutic preparations to be administered by untrained personnel or to be self-injected. Generally, these devices include a reservoir that is pre-filled with the liquid therapeutic preparation, and some type of automatic needle-injection mechanism that can be triggered by the user. When the volume of fluid or drug to be administered is generally below a certain volume, such as 1 mL, an auto-injector is typically used, which typically has an injection time of about 10 to 15 seconds. When the volume of fluid or drug to be administered is above 1 mL, the injection time generally becomes longer resulting in difficulties for the patient to maintain contact between the device and the target area of the patient's skin. Further, as the volume of drug to be administered becomes larger, increasing the time period for injection becomes desirable. The traditional method for a drug to be injected slowly into a patient is to initiate an IV and inject the drug into the patient's body slowly. Such a procedure is typically performed in a hospital or outpatient setting.

Certain devices allow for self-injection in a home setting and are capable of gradually injecting a liquid therapeutic preparation into the skin of a patient. In some cases, these devices are small enough (both in height and in overall size) to allow them to be "worn" by a patient while the liquid therapeutic preparation is being infused into the patient. These devices typically include a pump or other type of discharge mechanism to force the liquid therapeutic preparation to flow out of a reservoir and into the injection needle. Such devices also typically include a valve or flow control mechanism to cause the liquid therapeutic preparation to begin to flow at the proper time and a triggering mechanism to initiate the injection.

In WO 2015/191478 A1, liquid drug cartridges and an associated inhaler are used to deliver one more separate doses of an aerosolized liquid drug. In WO 2015/191478 A1, a cartridge includes a container for storing the liquid drug, an end cap having an ejection opening, a filter element, and a piston that is repositionable relative to the container to selectively eject a volume of liquid drug from the ejection opening. The filter element of WO 2015/191478 A1 filters the liquid drug prior to ejection from the ejection opening. The liquid drug cartridge of WO 2015/191478 A1 can be coupled with an inhaler that includes an aerosol generator. The aerosol generator of WO 2015/191478 A1 includes a vibratable membrane onto which the liquid drug is ejected. In WO 2015/191478 A1, the liquid drug is aerosolized by the vibration of the membrane for inhalation by a user.
CN 105 999 482 A, WO 2017/142808 A, US 2016/367767 A1, WO 2010/133698 A2, US 2005/047975 A1 AND US 2013/317422 A1

### DESCRIBE FURTHER RELEVANT STATE OF THE ART.

### SUMMARY OF THE INVENTION

The invention is defined in the independent claim. In one aspect, a drug delivery system for injecting a medicament includes a housing defining a cavity, a container received within the cavity and configured to receive a medicament, the container comprising a stopper configured to move within the container and a closure, a drive assembly received within the cavity and configured to drive the stopper within the container, a needle actuator assembly received within the cavity and comprising a needle configured to be placed in fluid communication with the container, the needle moveable from a first position and a second position spaced from the first position, and a sterilization arrangement received within the cavity and configured to sterilize at least one of the container, the drive assembly, and the needle actuator assembly upon activation of the drug delivery system.

In another aspect, the sterilization arrangement may include at least one light source that radiates ultraviolet light to sterilize at least one of the container, the drive assembly, and the needle actuator assembly. The at least one light source may include at least one light emitting diode (LED) light. The sterilization arrangement may include a power source operatively connected to the at least one light source. The power source may include at least one battery. The sterilization arrangement may include a sensor operatively connected to the power source and configured to send a signal to the power source upon initiation of a drug delivery process for the drug delivery system. An actuation button is provided in the housing and movable between a first, inactive position and a second, activation position in which a drug delivery process of the drug delivery system is initiated. The actuation button may include a magnet that activates the sensor when the actuation button is moved from the first, inactive position to the second, activation position. The sensor may be a Hall Effect transistor sensor. The sterilization arrangement may include a timer operatively connected to the light source and configured to shut off the light source after a predetermined amount of time. The at least one light source may include four light sources. The light sources may be positioned on different inner side surfaces of the housing.

### BRIEF DESCRIPTION OF THE DRAWINGS

The above-mentioned and other features and advantages of this disclosure, and the manner of attaining them, will become more apparent and the disclosure itself will be better understood by reference to the following descriptions of embodiments of the disclosure taken in conjunction with the accompanying drawings, wherein:
FIG. 1 is a perspective view of a drug delivery system according to one aspect of the present invention.
FIG. 2 is a perspective, cross-sectional view of the drug delivery system of FIG. 1 according to one aspect of the present invention.
FIG. 3 is a front, cross-sectional view of the drug delivery system of FIG. 1 according to one aspect of the present invention.
FIG. 4 is a top view of the drug delivery system of FIG. 1 according to one aspect of the present invention, showing a top portion of the housing removed and the drug delivery system in a pre-use position.
FIG. 5 is a top, cross-sectional view of the drug delivery system of FIG. 1 according to one aspect of the present invention, showing the drug delivery system in a pre-use position.
FIG. 6 is a front, cross-sectional view of the drug delivery system of FIG. 1 according to one aspect of the present invention, showing the drug delivery system in a pre-use position.
FIG. 7 is a top view of the drug delivery system of FIG. 1 according to one aspect of the present invention, showing a top portion of the housing removed and the drug delivery system in an initial actuation position.
FIG. 8 is a top, cross-sectional view of the drug delivery system of FIG. 1 according to one aspect of the present invention, showing the drug delivery system in an initial actuation position.
FIG. 9 is a front, cross-sectional view of the drug delivery system of FIG. 1 according to one aspect of the present invention, showing the drug delivery system in an initial actuation position.
FIG. 10 is a top view of the drug delivery system of FIG. 1 according to one aspect of the present invention, showing a top portion of the housing removed and the drug delivery system in a use position.
FIG. 11 is a top, cross-sectional view of the drug delivery system of FIG. 1 according to one aspect of the present invention, showing the drug delivery system in a use position.
FIG. 12 is a front, cross-sectional view of the drug delivery system of FIG. 1 according to one aspect of the present invention, showing the drug delivery system in a use position.
FIG. 13 is a top view of the drug delivery system of FIG. 1 according to one aspect of the present invention, showing a top portion of the housing removed and the drug delivery system in a post-use position.
FIG. 14 is a top, cross-sectional view of the drug delivery system of FIG. 1 according to one aspect of the present invention, showing the drug delivery system in a post-use position.
FIG. 15 is a front, cross-sectional view of the drug delivery system of FIG. 1 according to one aspect of the present invention, showing the drug delivery system in a post-use position.
FIG. 16 is a top view of a drug delivery system including a sterilization arrangement according to another aspect of the present invention, showing a top portion of the housing removed.
FIG. 17 is a schematic illustration of the sterilization arrangement of the drug delivery system of FIG. 16.

### DETAILED DESCRIPTION

The following description is provided to enable those skilled in the art to make and use the described embodiments contemplated for carrying out the invention. Various modifications, variations, and alternatives, however, will remain readily apparent to those skilled in the art.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

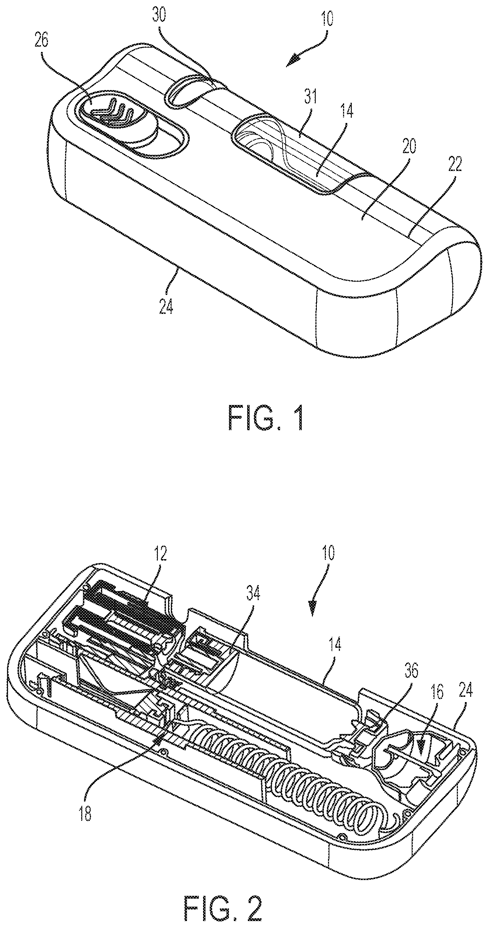

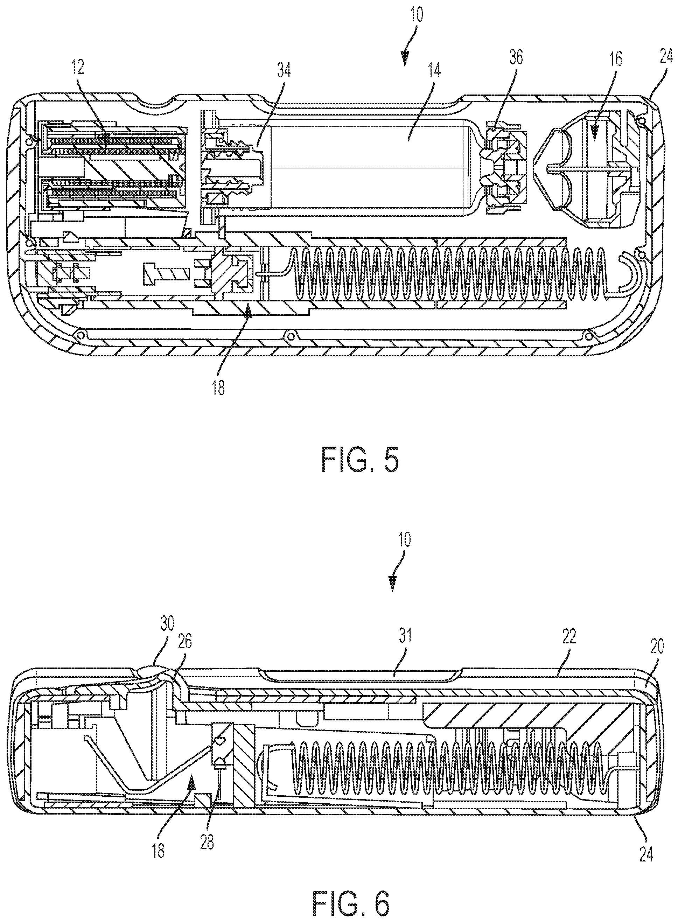

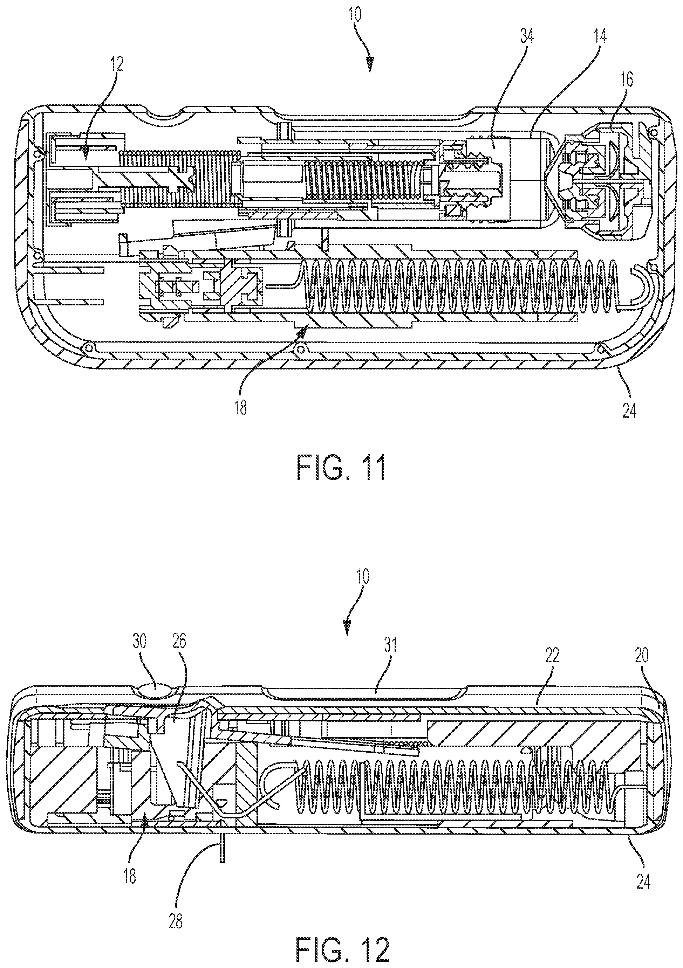

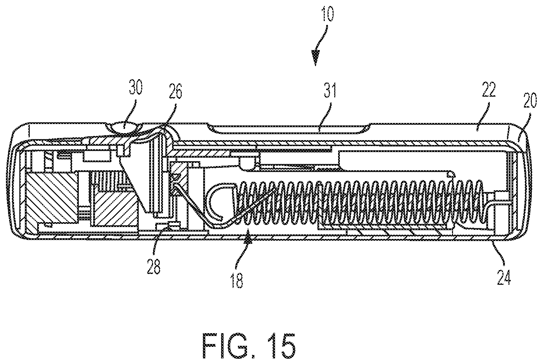

Referring to FIGS. 1-15, a drug delivery system 10 according to one aspect of the present disclosure includes a drive assembly 12, a container 14, a valve assembly 16, and a needle actuator assembly 18. The drive assembly 12, the container 14, the valve assembly 16, and the needle actuator assembly 18 are at least partially positioned within a cavity defined by a housing 20. The housing 20 includes a top portion 22 and a bottom portion 24, although other suitable arrangements for the housing 20 may be utilized. In one aspect, the drug delivery system 10 is an injector device configured to be worn or secured to a user and to deliver a predetermined dose of a medicament provided within the container 14 via injection into the user. The system 10 may be utilized to deliver a "bolus injection" where a medicament is delivered within a set time period. The medicament may be delivered over a time period of up to 45 minutes, although other suitable injection amounts and durations may be utilized. A bolus administration or delivery can be carried out with rate controlling or have no specific rate controlling. The system 10 may deliver the medicament at a fixed pressure to the user with the rate being variable. The general operation of the system 10 is described below in reference to FIGS. 1-15.

Referring again to FIGS. 1-15, the system 10 is configured to operate through the engagement of an actuation button 26 by a user, which results in a needle 28 of the needle assembly 18 piercing the skin of a user, the actuation of the drive assembly 12 to place the needle 28 in fluid communication with the container 14 and to expel fluid or medicament from the container 14, and the withdrawal of the needle 28 after injection of the medicament is complete. The general operation of a drug delivery system is shown and described in International Publication Nos. 2013/155153 and 2014/179774. The housing 20 of the system 10 includes an indicator window 30 for viewing an indicator arrangement 32 configured to provide an indication to a user on the status of the system 10 and a container window 31 for viewing the container 14. The indicator window 30 may be a magnifying lens for providing a clear view of the indicator arrangement 32. The indicator arrangement 32 moves along with the needle actuator assembly 18 during use of the system 10 to indicate a pre-use status, use status, and post-use status of the system 10. The indicator arrangement 32 provides visual indicia regarding the status, although other suitable indicia, such an auditory or tactile, may be provided as an alternative or additional indicia.

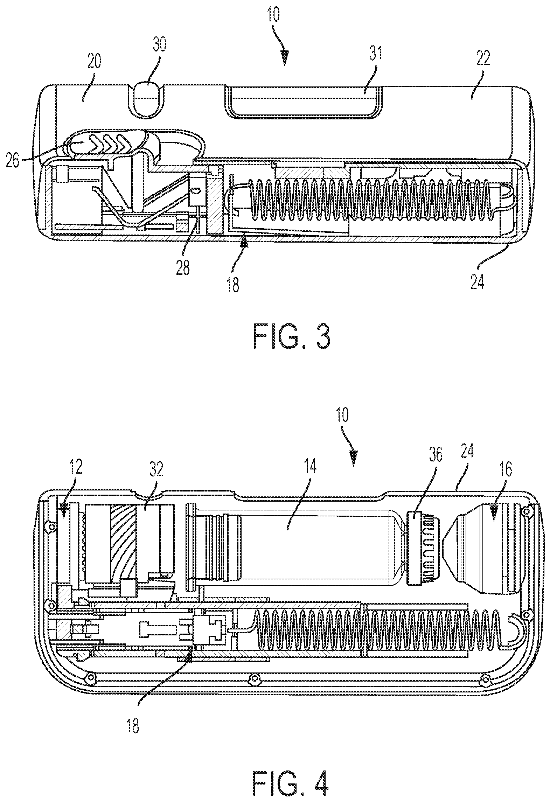

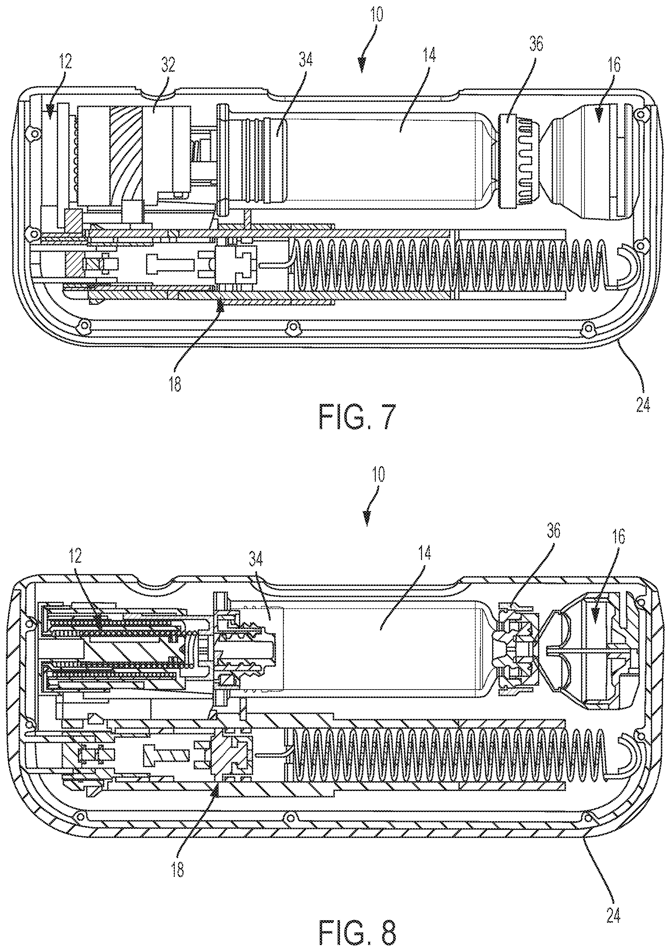

Referring to FIGS. 4-6, during a pre-use position of the system 10, the container 14 is spaced from the drive assembly 12 and the valve assembly 16 and the needle 28 is in a retracted position. During the initial actuation of the system 10, as shown in FIGS. 7-9, the drive assembly 12 engages the container 14 to move the container 14 toward the valve assembly 16, which is configured to pierce a closure 36 of the container 14 and place the medicament within the container 14 in fluid communication with the needle 28 via a tube (not shown) or other suitable arrangement. The drive assembly 12 is configured to engage a stopper 34 of the container 14, which will initially move the entire container 14 into engagement with the valve assembly 16 due to the incompressibility of the fluid or medicament within the container 14. The initial actuation of the system 10 is caused by engagement of the actuation button 26 by a user, which releases the needle actuator assembly 18 and the drive assembly 12 as discussed below in more detail. During the initial actuation, the needle 28 is still in the retracted position and about to move to the extended position to inject the user of the system 10.

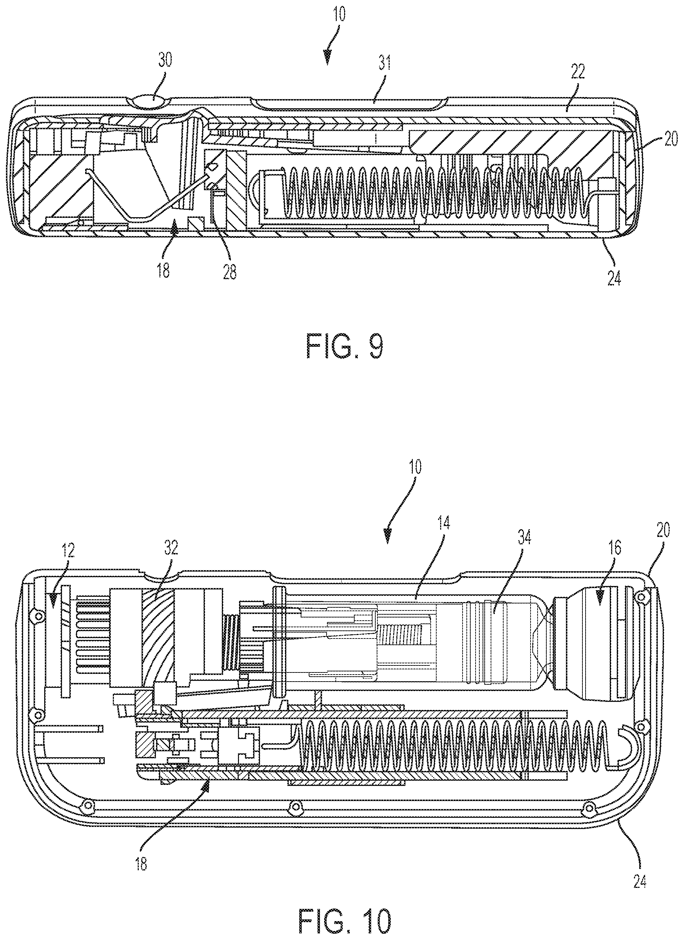

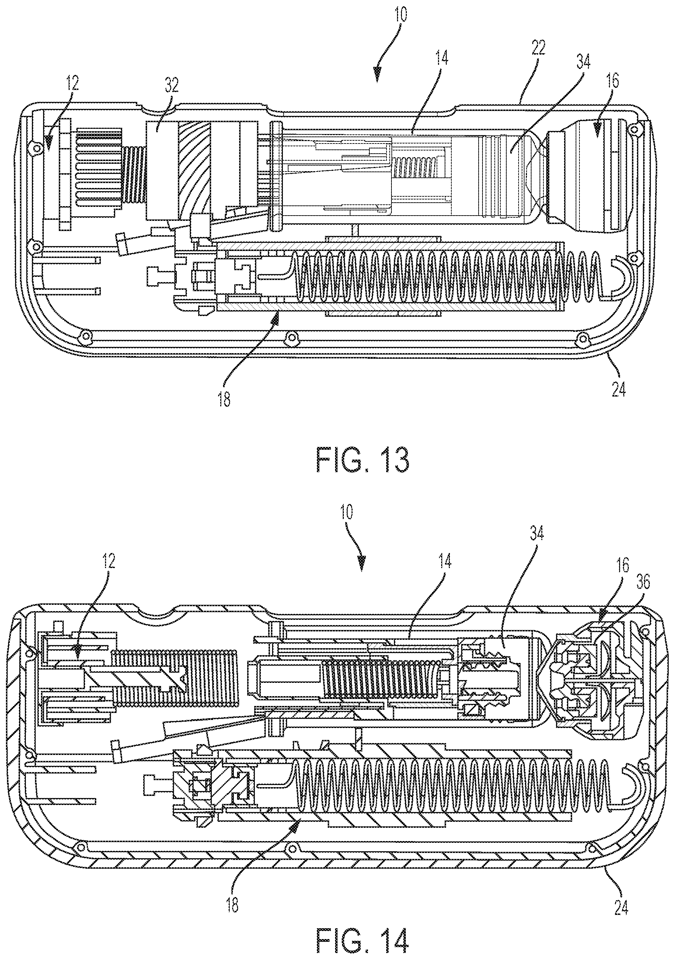

During the use position of the system 10, as shown in FIGS. 10-12, the needle 28 is in the extended position at least partially outside of the housing 20 with the drive assembly 12 moving the stopper 34 within the container 14 to deliver the medicament from the container 14, through the needle 28, and to the user. In the use position, the valve assembly 16 has already pierced a closure 36 of the container 14 to place the container 14 in fluid communication with the needle 28, which also allows the drive assembly 12 to move the stopper 34 relative to the container 14 since fluid is able to be dispensed from the container 14. At the post-use position of the system 10, shown in FIGS. 13-15, the needle 28 is in the retracted position and engaged with a pad to seal the needle 28 and prevent any residual flow of fluid or medicament from the container 14. The container 14 and valve assembly 16 may be the container 14 and valve assembly 16 shown and described in International Publication No. WO 2015/081337.

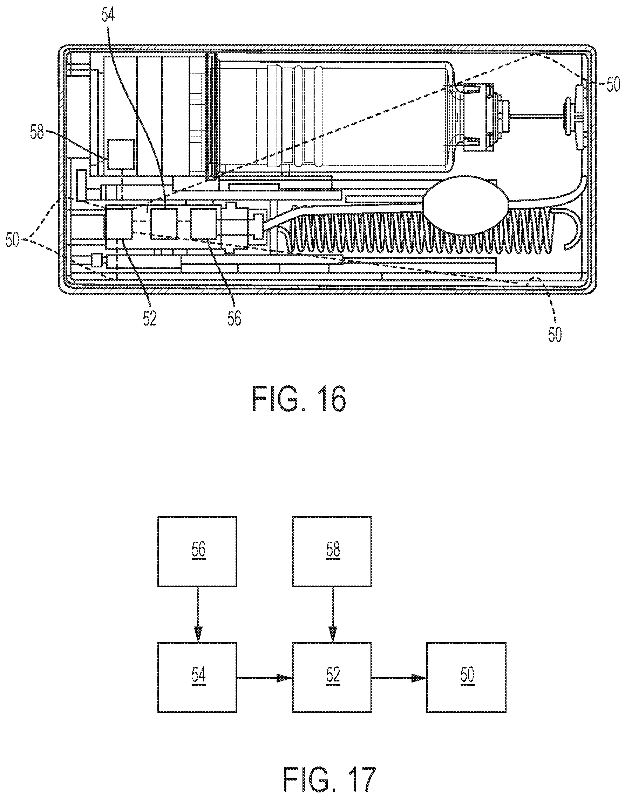

Referring to FIGS. 16 and 17, in one aspect, a sterilizer is also provided in the system 10. The sterilizer includes a light source 50 to sterilize the inner components of the system 10. In one aspect, the light source 50 provides ultraviolet (UV) light to the inner cavity of the housing 20. The UV light provided by the light source 50 is directed to the inner components of the system 10 to sterilize the inner components, such as the drive assembly 12, the container 14, the valve assembly 16, and the needle actuator assembly 18. In one aspect, the light source 50 includes one or more light emitting diode (LED) lights. In one aspect, a single light source 50 is provided in the housing 20. It is also contemplated that a plurality of light sources 50 are provided in the housing 20. In one aspect, four light sources 50 are provided in the housing 20. In one aspect, the light source 50 shines UV light on each component in the system 10. In another aspect, the light source 50 shines UV light on a portion of the components in the system 10. Any bacteria or contaminants growing or developing inside of the system 10 are sterilized by the UV light to provide a sterile environment inside the housing 20. In one aspect, the light sources 50 are each positioned on a different inner side surface of the housing 20.

Referring again to FIGS. 16 and 17, in one aspect, the light sources 50 are connected to a power source 52. The power source 52 may be any arrangement capable of providing power to illuminate the light sources 50. The power source 52 may be provided within the housing 20. In particular, the power source 52 may be provided on the top portion 22 and/or the bottom portion 24. In one aspect, the power source 52 includes at least one battery. It is also contemplated that the power source 52 may be provided as a power source external to the housing 20. For example, the power source 52 may be a battery pack wired to the housing 20, but provided external to the housing 20. The power source 52 may be replaceable and/or rechargeable. In one aspect, a plurality of batteries are provided as the power source 52 to power the light sources 50. The batteries may be charged by an external connector, such as a cord extending to an electrical outlet, or by an induction arrangement (not shown).

Referring again to FIGS. 16 and 17, a sensor 54 is also provided in the housing 20 to actuate the light sources 50 upon initiation of the drug delivery actuation process. The sensor 54 is configured to sense when the drug delivery actuation process has been initiated and sends a signal to the power source 52 to supply power from the power source 52 to the light sources 50 so that UV light is emitted by the light sources 50 to sterilize one or more components in the system 10. In one aspect, the sensor 54 is a Hall Effect transistor sensor that cooperates with a magnet 56 provided on or in a switch or button of the system 10. In one aspect, the sensor 54 is provided on the actuation button 26 of the system 10. Not according to the invention, it is also contemplated that the sensor 54 could be provided on a switch or button (not shown) separate from the actuation button 26 so that the light source 50 could be activated independently of the drug delivery actuation process. When the actuation button 26 of the system 10 is actuated, the sensor 54 sends a signal to activate the power source 52 to supply power to the light sources 50. Therefore, as the system 10 proceeds through the drug delivery process, the light sources 50 sterilize the inner components of the system 10. In another aspect, a timer 58 is connected to the power source 52 so that, after a predetermined amount of time of activation, the light sources 50 will be turned off. The length of time that the light sources 50 are activated is adjustable by the timer 58.

Referring to the device of FIG. 16 and the logic diagram of FIG. 17, a method of sterilizing a drug delivery system 10 is described. As described above, to actuate the drug delivery process, the actuation button 26 is depressed. As the actuation button 26 is depressed, the magnet 56 provided in the actuation button 26 actuates the sensor 54. The sensor 54 sends a signal to the power source 52 to activate the power source 52. The power source 52 then supplies power to the light sources 50 to activate the LED lights within the light sources 50. The LED lights of the light sources 50 cast UV light over the inner components of the system 10 to sterilize the inner components. In one aspect, the UV light is cast on at least one of the drive assembly 12, the container 14, the valve assembly 16, and the needle actuator assembly 18. It is also contemplated that the UV light may be cast on each of the drive assembly 12, the container 14, the valve assembly 16, and the needle actuator assembly 18. With the actuation button 26 having been depressed and the light sources 50 having been activated, after a predetermined amount of time, the timer 58 shuts off the light sources 50 to stop the emittance of the UV light. After use of the system 10, a user can recharge the power source 54 either internally or externally of the housing 20.

Elements of one disclosed aspect can be combined with elements of one or more other disclosed aspects to form different combinations, all of which are considered to be within the scope of the present invention.

While this disclosure has been described as having exemplary designs, the present disclosure can be further modified within the scope of this disclosure. This application is therefore intended to cover any variations, uses, or adaptations of the disclosure using its general principles. Further, this application is intended to cover such departures from the present disclosure as come within known or customary practice in the art to which this disclosure pertains and which fall within the limits of the appended claims.

## Claims

1. A drug delivery system (10) for injecting a medicament into a patient's skin wherein the system comprises a housing (20) defining a cavity,
a container (14) received within the cavity and configured to receive a medicament, the container (14) comprising a stopper (34) configured to move within the container,
a drive assembly (12) received within the cavity and configured to drive the stopper (34) within the container (14),
a needle actuator assembly (18) received within the cavity and comprising a needle (28) configured to be placed in fluid communication with the container (14), the needle (28) moveable from a first position to a second position spaced from the first position,
an activation button (26) configured to initiate drug delivery, the activation button (26) provided in the housing (20) and movable between an inactive position and an activation position in which a drug delivery process of the drug delivery system (10) is activated, and
a sterilizer received within the cavity and configured to sterilize at least one of the container (14), the drive assembly (12), and the needle actuator assembly (18) following activation of the drug delivery system (10) by actuation of the activation button.

2. The drug delivery system (10) as claimed in claim 1, wherein the sterilizer comprises at least one light source (50) that radiates ultraviolet light to sterilize at least one of the container (14), the drive assembly (12), and the needle actuator assembly (18).

3. The drug delivery system (10) as claimed in claim 2, wherein the at least one light source (50) includes at least one light emitting diode (LED) light.

4. The drug delivery system (10) as claimed in claim 2, wherein the sterilizer further comprises a power source (52) operatively connected to the at least one light source (50).

5. The drug delivery system (10) as claimed in claim 4, wherein the power source (52) comprises at least one battery.

6. The drug delivery system (10) as claimed in claim 4, wherein the sterilizer further comprises a sensor (54) operatively connected to the power source (52) and configured to send a signal to the power source (52) upon initiation of a drug delivery process for the drug delivery system (10).

7. The drug delivery system (10) as claimed in claim 6, wherein the actuation button (26) comprising a magnet that activates the sensor (54) when the actuation button (26) is moved from the first, inactive position to the second, activation position.

8. The drug delivery system (10) as claimed in claim 6, wherein the sensor (54) is a Hall Effect transistor sensor.

9. The drug delivery system (10) as claimed in claim 2, wherein the sterilizer further comprises a timer (58) operatively connected to the light source and configured to shut off the light source (50) after a predetermined amount of time.

10. The drug delivery system (10) as claimed in claim 2,
wherein the at least one light source (50) comprises four light sources, and
wherein the light sources are positioned on different inner side surfaces of the housing (20).

## Patentansprüche

1. Medikamentenabgabesystem (10) zum Injizieren eines Medikaments in die Haut eines Patienten, wobei das System Folgendes aufweist:
ein Gehäuse (20), das einen Hohlraum definiert,
einen Behälter (14), der in dem Hohlraum aufgenommen und zur Aufnahme eines Medikaments ausgebildet ist, wobei der Behälter (14) einen Stopfen (34) aufweist, der dazu ausgebildet ist, sich innerhalb des Behälters zu bewegen,
eine Antriebsanordnung (12), die in dem Hohlraum aufgenommen und dazu ausgebildet ist, den Stopfen (34) innerhalb des Behälters (14) anzutreiben,
eine Nadelbetätigungsanordnung (18), die in dem Hohlraum aufgenommen ist und eine Nadel (28) aufweist, die dazu ausgebildet ist, in Fluidverbindung mit dem Behälter (14) gebracht zu werden, wobei die Nadel (28) aus einer ersten Position in eine von der ersten Position beabstandete zweite Position bewegbar ist,
einen Betätigungsknopf (26), der dazu ausgebildet ist, eine Medikamentenabgabe einzuleiten, wobei der Betätigungsknopf (26) in dem Gehäuse (20) vorgesehen und zwischen einer inaktiven Position und einer Aktivierungsposition bewegbar ist, in der ein Medikamentenabgabevorgang des Medikamentenabgabesystems (10) aktiviert wird, und
eine Sterilisationsanordnung, die in dem Hohlraum aufgenommen und dazu ausgebildet ist, nach Aktivierung des Medikamentenabgabesystems (10) durch Betätigung des Betätigungsknopfs mindestens eine der folgenden Komponenten zu sterilisieren: den Behälter (14), die Antriebsanordnung (12) und die Nadelbetätigungsanordnung (18).

2. Medikamentenabgabesystem (10) nach Anspruch 1, wobei die Sterilisationsanordnung mindestens eine Lichtquelle (50) aufweist, die ultraviolettes Licht abstrahlt, um mindestens eine der folgenden Komponenten zu sterilisieren: den Behälter (14), die Antriebsanordnung (12) und die Nadelbetätigungsanordnung (18).

3. Medikamentenabgabesystem (10) nach Anspruch 2, wobei die mindestens eine Lichtquelle (50) mindestens eine Leuchtdiode (LED) aufweist.

4. Medikamentenabgabesystem (10) nach Anspruch 2, wobei die Sterilisationsanordnung ferner eine Stromquelle (52) aufweist, die mit der mindestens einen Lichtquelle (50) wirkverbunden ist.

5. Medikamentenabgabesystem (10) nach Anspruch 4, wobei die Stromquelle (52) mindestens eine Batterie aufweist.

6. Medikamentenabgabesystem (10) nach Anspruch 4, wobei die Sterilisationsanordnung ferner einen Sensor (54) aufweist, der mit der Stromquelle (52) wirkverbunden und dazu ausgebildet ist, bei Einleitung eines Medikamentenabgabevorgangs des Medikamentenabgabesystems (10) ein Signal an die Stromquelle (52) zu senden.

7. Medikamentenabgabesystem (10) nach Anspruch 6, wobei der Betätigungsknopf (26) einen Magneten aufweist, der den Sensor (54) aktiviert, wenn der Betätigungsknopf (26) aus der ersten, inaktiven Position in die zweite Aktivierungsposition bewegt wird.

8. Medikamentenabgabesystem (10) nach Anspruch 6, wobei der Sensor (54) ein Hall-Effekt-Transistorsensor ist.

9. Medikamentenabgabesystem (10) nach Anspruch 2, wobei die Sterilisationsanordnung ferner einen Timer (58) aufweist, der mit der Lichtquelle wirkverbunden und dazu ausgebildet ist, die Lichtquelle (50) nach einer vorbestimmten Zeitspanne auszuschalten.

10. Medikamentenabgabesystem (10) nach Anspruch 2, wobei die mindestens eine Lichtquelle (50) vier Lichtquellen aufweist und wobei die Lichtquellen an verschiedenen inneren Seitenflächen des Gehäuses (20) positioniert sind.

## Revendications

1. Système d'administration de médicament (10) pour injecter un médicament dans la peau d'un patient dans lequel le système comprend un logement (20) définissant une cavité,
un récipient (14) reçu à l'intérieur de la cavité et configuré pour recevoir un médicament, le récipient (14) comprenant un piston (34) configuré pour se déplacer à l'intérieur du récipient,
un ensemble d'entraînement (12) reçu à l'intérieur de la cavité et configuré pour entraîner le piston (34) à l'intérieur du récipient (14),
un ensemble actionneur d'aiguille (18) reçu à l'intérieur de la cavité et comprenant une aiguille (28) configurée pour être placée en communication fluidique avec le récipient (14), l'aiguille (28) étant mobile d'une première position à une seconde position espacée de la première position,
un bouton d'activation (26) configuré pour initier l'administration de médicament, le bouton d'activation (26) étant prévu dans le logement (20) et mobile entre une position inactive et une position d'activation dans laquelle un processus d'administration de médicament du système d'administration de médicament (10) est activé, et
un stérilisateur reçu dans la cavité et configuré pour stériliser au moins l'un parmi le récipient (14), l'ensemble d'entraînement (12) et l'ensemble actionneur d'aiguille (18) après activation du système d'administration de médicament (10) par actionnement du bouton d'activation.

2. Système d'administration de médicament (10) selon la revendication 1, dans lequel le stérilisateur comprend au moins une source de lumière (50) qui rayonne de la lumière ultraviolette pour stériliser au moins l'un parmi le récipient (14), l'ensemble d'entraînement (12) et l'ensemble actionneur d'aiguille (18).

3. Système d'administration de médicament (10) selon la revendication 2, dans lequel l'au moins une source de lumière (50) inclut au moins une lumière à diode électroluminescente (LED).

4. Système d'administration de médicament (10) selon la revendication 2, dans lequel le stérilisateur comprend en outre une source d'alimentation (52) connectée de manière fonctionnelle à l'au moins une source de lumière (50).

5. Système d'administration de médicament (10) selon la revendication 4, dans lequel la source d'alimentation (52) comprend au moins une batterie.

6. Système d'administration de médicament (10) selon la revendication 4, dans lequel le stérilisateur comprend en outre un capteur (54) connecté de manière fonctionnelle à la source d'alimentation (52) et configuré pour envoyer un signal à la source d'alimentation (52) lors de l'initiation d'un processus d'administration de médicament pour le système d'administration de médicament (10).

7. Système d'administration de médicament (10) selon la revendication 6, dans lequel le bouton d'activation (26) comprend un aimant qui active le capteur (54) lorsque le bouton d'activation (26) est déplacé de la première position inactive à la seconde position d'activation.

8. Système d'administration de médicament (10) selon la revendication 6, dans lequel le capteur (54) est un capteur à transistor à effet Hall.

9. Système d'administration de médicament (10) selon la revendication 2, dans lequel le stérilisateur comprend en outre une minuterie (58) connecté de manière fonctionnelle à la source de lumière et configuré pour éteindre la source de lumière (50) après une durée prédéterminée.

10. Système d'administration de médicament (10) selon la revendication 2,
dans lequel l'au moins une source de lumière (50) comprend quatre sources de lumière, et
dans lequel les sources de lumière sont positionnées sur différentes surfaces latérales intérieures du logement (20).
